# EUROPÄISCHE PATENTANMELDUNG

(11) **EP 0 965 642 A1**
(43) Veröffentlichungstag der Anmeldung: **22.12.1999**
(21) Anmeldenummer: 99108669.5
(22) Anmeldetag: 17.05.1999
(51) Int. Cl.: C12N 15/63, C12N 15/10, C12N 15/70

(54) **Klonierungsvektor, seine Herstellung und Verwendung zur Analyse von mRNA Expressionsmuster**

(30) Priorität: 18.05.1998 DE 19822287
(71) Anmelder: Switch Biotech GmbH, 82152 Martinsried (DE)
(72) Erfinder: Halle, Jörn-Peter, Dr., 82377 Penzberg (DE); Regenbogen, Johannes, Dr., 82152 Martinsried (DE); Goppelt, Andreas, Dr., 80636 München (DE)
(74) Vertreter: Bösl, Raphael, Dr. rer. nat., Dipl.-Chem.

(57) **Zusammenfassung**

Die Erfindung betrifft einen Klonierungsvektor enthaltend
(a) eine Klonierungsstelle, die die Klonierung einer Nukleinsäure in definierter Orientierung erlaubt,
(b) mindestens eine Schnittstelle, die zu der Klonierungsstelle (a) benachbart ist und die in Nukleinsäuren nur selten vorhanden ist,
(c) einen langen Bereich, der an der bezogen auf die Schnittstelle (b) gegenüberliegenden Seite der Klonierungsstelle (a) angeordnet ist, wobei der lange Bereich und der Bereich zwischen der Klonierungsstelle (a) und der Schnittstelle (b) weder die Klonierungsstelle noch mindestens zwei Schnittstellen, die in Nukleinsäuren häufig vorhanden sind, enthält.

SEQUENZPROTOKOLL

<110> Switch Biotech GmbH

<120> Klonierungsvektor, seine Herstellung und Verwendung zur Analyse von
mRNA Expressionsmuster

<150> DE 198 22 287.4
<151> 1998-05-18

<160> 18

<170> Word 6.0, PC-DOS/MS-DOS

<210> 1
<211> 33
<212> DNA
<213> künstliche Sequenz

<400> 1
GAGAGAGAGA CTCGAGTTTT TTTTTTTTTT TTT 33

<210> 2
<211> 13
<212> DNA
<213> künstliche Sequenz

<400> 2
GGCCAGGGTG GCC 13

<210> 3
<211> 24
<212> DNA
<213> künstliche Sequenz

<400> 3
CTCGAGGCCA GGGTGGCCGA TCGA 24

<210> 4
<211> 13
<212> DNA
<213> künstliche Sequenz

<400> 4
CTCGAGGCCA GGG 13

<210> 5
<211> 14
<212> DNA
<213> künstliche Sequenz

<400> 5
TCGATCGGCC ACCC 14

<210> 6
<211> 13
<212> DNA
<213> künstliche Sequenz

<400> 6
CCCTGGCCTC GAG 13

<210> 7
<211> 13
<212> DNA
<213> künstliche Sequenz

<400> 7
CCCTGGCCTC GAG 13

<210> 8
<211> 71
<212> DNA
<213> künstliche Sequenz

<400> 8
AGCTTGGCGC GCCGAATTCT ATCTCGAGCG GCCGCAGCTG AGATCGTACC
50
CTATAGTGAG TCGTATTACG T 71

<210> 9
<211> 63
<212> DNA
<213> künstliche Sequenz

<400> 9
AATACGACTC ACTATAGGGT ACGATCTCAG CTGCGGCCGC TCGAGATAGAA
50
TTCGGCGCG CCA 63

<210> 10
<211> 10
<212> DNA

<213>  künstliche Sequenz

<400>  10
AGGCGCGCCT                                                                10

<210>  11
<211>  14
<212>  DNA
<213>  künstliche Sequenz

<400>  11
GGCGCGCCTT  GAGT                                                          14

<210>  12
<211>  13
<212>  DNA
<213>  künstliche Sequenz

<400>  12
GGGAAGGCGC  GCC                                                           13

<210>  13
<211>  31
<212>  DNA
<213>  künstliche Sequenz

<400>  13
CCCCAAGCTT GTGAATATAT CGAACAGTCA G                                        31

<210>  14

<211>  24
<212>  DNA
<213>  künstliche Sequenz

<400>  14
CCGGCGCGCC  TCCCGGTCTT  TTCG                                                    24

<210>  15
<211>  11
<212>  DNA
<213>  künstliche Sequenz

<400>  15
AAGCTTGTGA  A                                                                   11

<210>  16
<211>  13
<212>  DNA
<213>  künstliche Sequenz

<400>  16
CGGGAGGCGC GCC                                                                  13

<210>  17
<211>  48
<212>  DNA
<213>  künstliche Sequenz

<400>  17
GAGAGAGAGA  GAGAGAGAGA  ACTAGTCTCG  AGTTTTTTTT  TTTTTTTT   48

<210> 18
<211> 17
<212> DNA
<213> künstliche Sequenz

<400> 18
CAGGAGATGC TGTTCGT 17

## Beschreibung

Die vorliegende Erfindung betrifft einen Klonierungsvektor enthaltend
(a) eine Klonierungsstelle, die die Klonierung einer Nukleinsäure in definierter Orientierung erlaubt,
(b) mindestens eine Schnittstelle, die zu der Klonierungsstelle (a) benachbart ist und die in Nukleinsäuren nur selten vorhanden ist,
(c) einen langen Bereich, der an der bezogen auf die Schnittstelle (b) gegenüberliegenden Seite der Klonierungsstelle (a) angeordnet ist, wobei der lange Bereich und der Bereich zwischen der Klonierungsstelle (a) und der Schnittstelle (b) weder die Klonierungsstelle noch mindestens zwei Schnittstellen, die in Nukleinsäuren häufig vorhanden sind, enthält.

Der Vergleich von mRNA-Expressionsmuster zwischen verschiedenen Zellen bzw. Geweben spielt eine zunehmende Rolle in der biomedizinischen Forschung (siehe z. B. Adams, 1991, Science *252*, 1651-6). So wird beispielsweise aus dem Vergleich zwischen gesundem und erkranktem Gewebe auf mögliche Fehlregulationen geschlossen. Desweiteren erlaubt ein Vergleich zwischen pharmazeutisch behandeltem und nicht behandeltem Gewebe oder Zellen bzw. Kontrolltieren einen Rückschluß auf Wirkmechanismen von Pharmazeutika. Vergleiche zwischen verschiedenen Geweben bzw. Zelltypen erlauben auch die Identifikation von Differenzierungs- bzw. Kontrollgenen.

Um das mRNA-Expressionsmuster darzustellen, sind verschiedene Methoden entwickelt worden, die jedoch alle bestimmte Nachteile haben. So erfassen Methoden, die auf subtraktiven cDNA- Bibliotheken beruhen (Akopian und Wood, 1995, J. Biol. Chem. *270*, 21264-70; Deleersnijder et al., 1996, J. Biol. Chem. *271*, 19475-82; Diatchenko et al., 1996, Proc. Natl. Acad. Sci. U S A *93*, 6025-30; Gurskaya et al., 1996, Anal. Biochem. *240*, 90-7; Hubank und Schatz, 1994, Nucleic Acids Res. *22*, 5640-8; Lisitsyn und Wigler, 1993, Science *259*, 946-51; Yang und Sytkowski, 1996, Anal. Biochem. *237*, 109-14; Zeng et al., 1994, Nucleic Acids Res. *22*, 4381-5), nur große Differenzen im Expressionsmuster. Techniken, die auf der 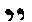 Differential Display RT-PCR" (Liang et al., 1992, Cancer Res. *52*, 6966-8; Liang und Pardee, 1992, Science *257*, 967-71) und deren Weiterentwicklungen (Prashar und Weissman, 1996, Proc. Natl. Acad. Sci. U S A *93*, 659-63; U.S. Patent No. 5, 459, 037) beruhen, können nur einen beschränkten Teil aller Gene analysieren und sind sehr zeitintensiv sowie fehleranfällig.

Der 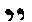 expressed sequence tag" (EST) Ansatz (Adams et al., 1992, Nature *355*, 632-4; Adams et al., 1991, Science *252*, 1651-6) analysiert das Expressionsmuster mit Hilfe der Sequenzierung vieler Klone von cDNA-Bibliotheken. Schon kurze Sequenzen des 3'-cDNA Endes (Marker oder 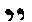 tag") erlauben die eindeutige Identifizierung des Gens. Unterschiedliche Häufigkeiten von cDNAs in verschiedenen Bibliotheken lassen zudem Rückschlüsse auf eine veränderte Genexpression zu. Obwohl dieser Ansatz sehr genaue quantitative Aussagen zuläßt, ist er sehr arbeitsintensiv. Weiterentwicklungen dieser Methode konzentrieren sich daher in erster Linie auf die Erhöhung des Durchsatzes mittels serieller (Velculescu et al., 1995, Science *270*, 484-7; Velculescu et al., 1997, Cell *88*, 243-51) oder paralleler (Brenner und Livak, 1989, Proc. Natl. Acad. Sci. U S A *86*, 8902-6.; U.S. Patent No. 5, 714, 330) Sequenzierung vieler kurzer Marker.

Beispielsweise beschreibt U.S. Patent No. 5,695,937 die serielle Analyse der Genexpression (SAGE), worin zuerst kurze cDNA-Sequenzen von mRNAs hergestellt werden, diese anschließend dimerisiert und multimerisiert und nach der Klonierung manuell sequenziert werden. Nachteil dieser Methode ist, daß nur ein kleiner Teil (<20 bp) der cDNA kloniert und über Sequenzierung identifiziert werden kann.

U.S. Patent No. 5,459,037 beschreibt ein Verfahren für eine simultane Sequenz-spezifische Identifizierung von mRNAs in einer mRNA-Population, worin für die Synthese von korrespondierenden cDNAs ein Primer-Gemisch verwendet wird, anschließend die cDNAs mit Hilfe von RNA-Polymerasen wiederum in cRNAs umgeschrieben werden und danach eine PCR durchgeführt wird. Die Analyse des Expressionsmusters erfolgt durch einen Vergleich der Intensitäten der Banden. Nachteil dieses Verfahrens ist, daß der PCR-Schritt häufig zu fehlerhaften Ergebnissen führt.

U.S. Patent No. 5,712,126 beschreibt die selektive PCR-Amplifikation der 3'-Enden von cDNA-Fragmenten, worin jedoch kein Primer-Gemisch, sondern 12 verschiedene cDNA-Synthesen durchgeführt werden und somit zusätzlich entsprechend aufwendig ist. Auch erfolgt die Analyse der Expressionsmuster durch Vergleich der Intensitäten der Banden mit entsprechender Fehlerbreite.

Ein weiteres Problem bei der Analyse von Genexpressionsmustern ist, daß cDNA Bibliotheken im allgemeinen einen hohen Prozentsatz von Klonen enthalten, die keine oder nur unvollständige cDNAs enthalten. Diese verringern den Analysedurchsatz und können das Analyseergebnis verfälschen.

Aufgabe der vorliegenden Erfindung war daher, ein Verfahren bereitzustellen, das die oben beschriebenen Nachteile vermeidet, insbesondere bei dem auf eine zusätzliche Sequenzierung der cDNA verzichtet werden kann, um so einen kostengünstigen und hohen Analysedurchsatz zu ermöglichen. Zudem soll auf die Polymerase-Kettenreaktion (PCR) verzichtet werden können, denn ein PCR-Schritt führt häufig zu fehlerhaften Ergebnissen.

Gegenstand der vorliegenden Erfindung ist daher ein Klonierungsvektor enthaltend
(a) eine Klonierungsstelle, die die Klonierung einer Nukleinsäure in definierter Orientierung erlaubt,
(b) mindestens eine Schnittstelle, die zu der Klonierungsstelle (a) benachbart ist und die in Nukleinsäuren nur selten vorhanden ist,
(c) einen langen Bereich, der an der bezogen auf die Schnittstelle (b) gegenüberliegenden Seite der Klonierungsstelle (a) angeordnet ist, wobei der lange Bereich und der Bereich zwischen der Klonierungsstelle (a) und der Schnittstelle (b) weder die Klonierungsstelle noch mindestens zwei Schnittstellen, die in Nukleinsäuren häufig vorhanden sind, enthält.

In einer bevorzugten Ausführungsform ist der lange Bereich länger als die Fragmente, die durch Schneiden mit Restriktionsnucleasen erhältlich sind, welche häufig vorhandene Schnittstellen erkennen.

In einer weiteren Ausführungsform enthält der Klonierungsvektor an der anderen Seite der Klonierungsstelle einen kurzen Bereich mit mehreren verschiedenen Schnittstellen, die in Nukleinsäuren häufig und in dem genannten langen Bereich nicht vorhanden sind.

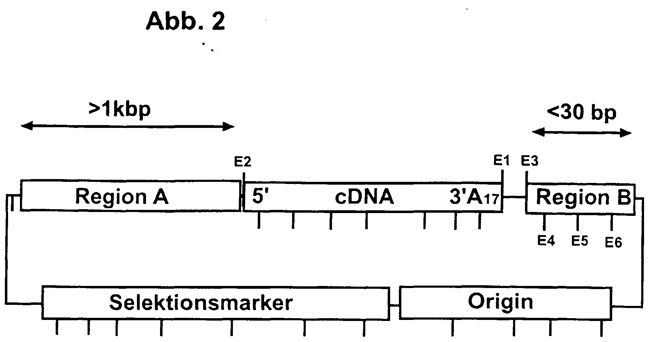

In einer bevorzugten Ausführungsform enthält die Klonierungsstelle zwei verschiedene Schnittstellen. Ein Beispiel eines erfindungsgemäßen Klonierungsvektors ist in Abb. 2 dargestellt.

Gemäß der vorliegenden Erfindung versteht man unter einer Schnittstelle, die in Nukleinsäuren häufig vorhanden ist, eine Stelle, die von Restriktionsendonucleasen auch Restriktionsenzyme genannt, mit einer Erkennungssequenz von höchstens einschließlich 4 Nukleotiden erkannt wird.

Beispiele derartiger Restriktionsendonucleasen sind AciI, AluI, BfaI, BsaJI, BslI, BscFI, BssKI, BstUI, Cac8I, CfoI, Csp6I, CviJI, DdeI, DpnI, DpnII, FmuI, Fnu4HI, HaeIII, HhaI, HinfI, HinPI, HpaII, MaeII, MaeIII, MboI, MnlI, MseI, MspI, MwoI, NlaIII, NlaIV, RsaI, Sau3AI, Sau96I, ScrFI, TaiI, TaqI, Tsp4CI oder Tsp509I, die alle erhältlich sind.

Unter einer Klonierungsstelle und Schnittstelle, die in Nukleinsäuren nur selten vorhanden ist, versteht man gemäß der vorliegenden Erfindung unabhängig voneinander eine Stelle, die von Restriktionsendonucleasen mit einer Erkennungssequenz von mindestens einschließlich 5 Nukleotiden, vorzugsweise mindestens einschließlich 6 Nukleotiden, die vor allem selten vorkommende Nukleotidkombinationen wie CG enthalten, insbesondere mindestens einschließlich 8 Nukleotiden erkannt wird.

Beispiele von Restriktionsendonucleasen mit einer oder mehreren Erkennungssequenzen von 5 Nukleotiden, sind AclWI, Alw26I, AlwI, AsuHPI, AvaII, BbvI, BccI, BcefI, BinI, BsbI, BscGI, Bse1I, BseNI, BsmAI, BsmFI, BspLU11III, BsrI, BsrSI, Bst71I, BstF5I, BstNI, CjeI, CjePI, EcoRII, FauI, FinI, FokI, HgaI, HphI, MboII, NciI, PleI, SfaNI, SimI, TauI, TfiI, TseI, Tsp45I, TspRI oder Vpa11AI, die alle erhältlich sind.

Beispiele von Restriktionsendonucleasen mit mindestens einer Erkennungssequenz von 6 Nukleotiden sind AccI, AflIII, ApoI, AvaI, BanI, BanII, BmgI, BsaI, BsaHI, BsaWI, BsiEI, BsiHKAI, BsoBI, Bsp1286I, BsrFI, BstYI, DsaI, EaeI, EcoO109I, GdiII, HaeI, HaeII, Hin4I, HincII, MmeI, MslI, MspA1I, NspI, SfcI, StyI, TatI, Tth111II, AatI, Acc113I, Acc65I, AcINI, AfIII, Alw44I, ApaI, ApaLI, AseI, Asp718I, AvrII, BalI, BamHI, BbuI, BbsI, BclI, BfrI, BglI, BglII, BlnI, BpiI, BpmI, BsaI, BsaMI, BseRI, BsmBI, BsmI, Bsp120I, Bsp1407I, Bsp19I, BspHI, BspLU11I, BspMI, BspTI, BsrGI, Bst1107I, Bst98I, DraI, Eam1104I, EarI, Ecl136II, Eco147I, Eco255I, Eco57I, EcoNI, EcoRI, EcoRV, EcoT22I, HindIII, HpaI, KpnI, MfeI, MscI, NcoI, NdeI, NheI, NsiI, PstI, PvuII, SacI, ScaI, SpeI, SphI, SspI, SstI, StuI oder XbaI.

Beispiele von Restriktionsendonucheasen, die eine Erkennungssequenz von 6 Nukleotiden, die selten vorkommende Nukleotidkombinationen wie CG enthalten, erkennen sind AatII, BbeI, BsiI, BsiWI, BsmBI, BspDI, BsrBI, BssHII, Bst2BI, BstBI, ClaI, EagI, EciI, Eco47III, EheI, Esp3I, FspI, KasI, MluI, NarI, NruI, Pfl1108I, PmlI, Psp1406I, PvuI, SacII, SalI, SnaBI oder XhoI.

Beispiele von Restriktionsendonucleasen, die eine Erkennungssequenz, die größer als 6 Nukleotide ist, erkennen, sind AscI, BaeI, FseI, NotI, PacI, PmeI, PpuMI, RsrII, SanDI, SapI, SexAI, SfiI, SgfI, SgrAI, SrfI, Sse8387I, SwaI, I-CeuI, PI-PspI, I-PpoI, PI-TliI oder PI-SceI.

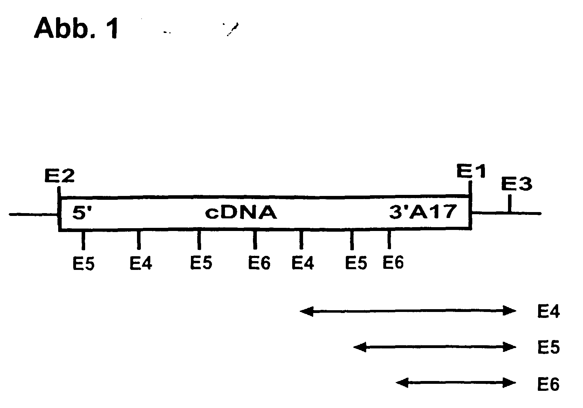

Mit Hilfe des erfindungsgemäßen Klonierungsvektors erfolgt beispielsweise die Identifizierung eines cDNA-Klons aufgrund des charakteristischen Abstands von Restriktionsschnittstellen zum 3'-Ende der cDNA (siehe z. B. Abb. 1). Da dieser Abstand bei verschiedenen Genen für ein gegebenes Restriktionsenzym identisch sein kann, erlaubt eine Analyse der DNA-Fragmentlängen oder DNA-Massen des 3'-Endes der cDNA, die durch mindestens zwei verschiedenen Restriktionsenzyme erzeugt werden, eine eindeutige Identifizierung. Die Fragmente der cDNA, die im Verlauf des Verfahrens markiert werden, umfassen vorzugsweise Teile des 3'-poly-A-Schwanzes, die cDNA bis zur nächsten 5'-gelegenen Restriktionsschnittstelle, sowie kurze Vektorsequenzen (siehe Abb.1).

Daher ist gemäß der vorliegenden Erfindung der genannte lange Bereich vorzugsweise länger als die Fragmente, die durch Schneiden von cDNAs mit Restriktionsendonucleasen erhältlich sind, die in Nukleinsäuren häufig vorhandene Schnittstellen erkennen. Insbesondere ist der lange Bereich länger als ca. 500 Nukleotide, vor allem länger als ca. 1000 Nukleotide. Der genannte kurze Bereich ist gemäß der weiteren Ausführungsform der vorliegenden Erfindung vorzugsweise kleiner als die Länge der Nukleinsäure, die sich von der Schnittstelle (b) bis zur ersten möglichen Schnittstelle in der einzuklonierenden Nukleinsäure, die in Nukleinsäuren häufig vorhanden ist, bzw. kleiner als die Länge der Nukleinsäure, die sich von der Schnittstelle (b) bis vorzugsweise zum Anfang des poly(A)-Schwanzes der einzuklonierenden cDNA erstreckt. Insbesondere ist der kurze Bereich kleiner als ca. 100 Nukleotide, vor allem kleiner als ca. 30 Nukleotide.

Alternativ kann der kurze Bereich entfallen, wenn gemäß dem Beispiel der vorliegenden Erfindung durch die Wahl der Erkennungsstelle E3 gewährleistet wird, daß nur die E3-E5 (bzw. E3-E4 und E3-E6) Fragmente, die das 3 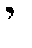 -Ende der cDNA enthalten, markiert werden, nicht aber die entsprechenden Fragmente des Vektors.

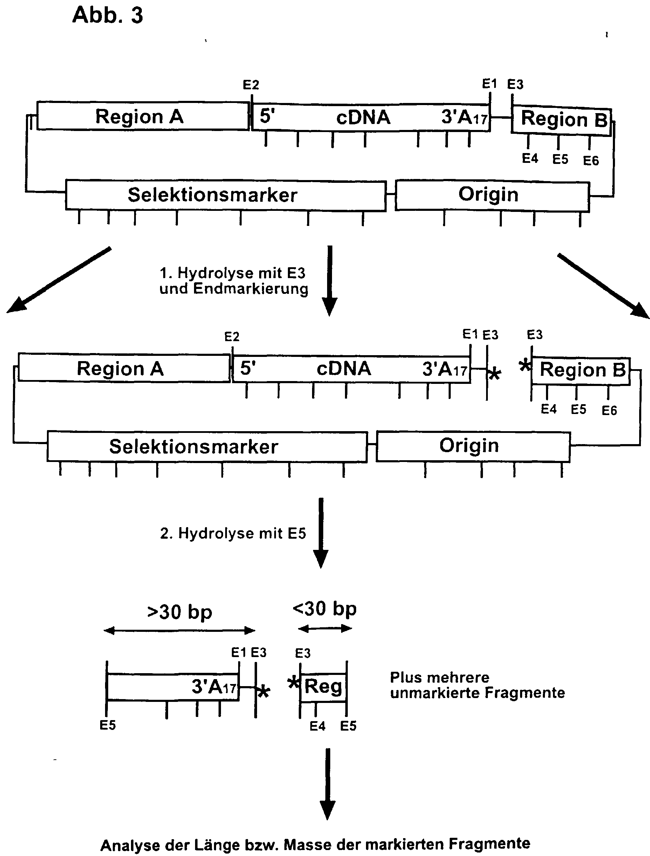

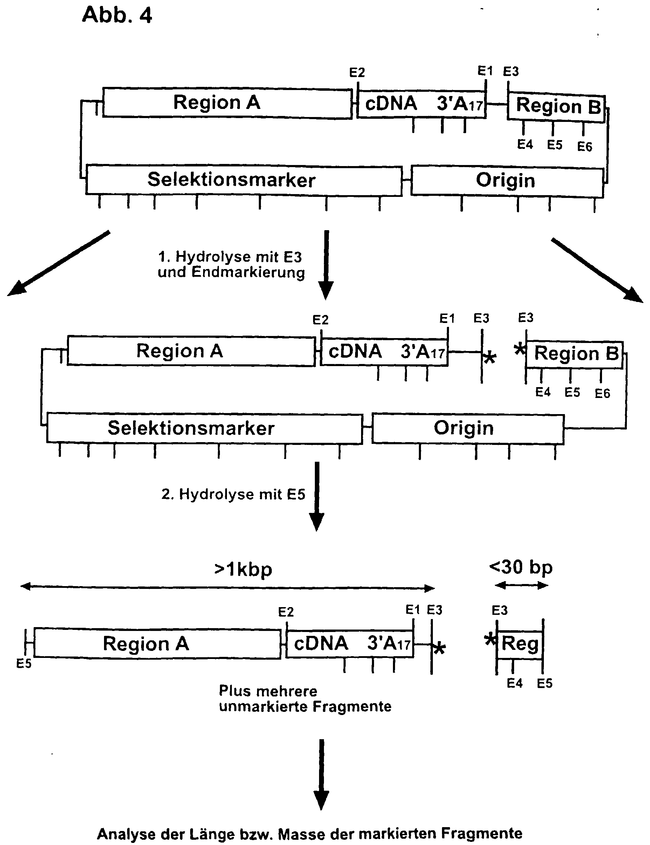

Ein besonders bevorzugter erfindungsgemäßer Vektor hat im allgemeinen folgende Eigenschaften (siehe auch Abb. 2):
1. Er enthält eine Insertionsstelle für die cDNA mit den Erkennungsstellen der Restriktionsenzyme E1 und E2, die eine gerichtete Klonierung der cDNA ermöglichen. Die Erkennungsstellen für die Enzyme E1 und E2 kommen im allgemeinen nur einmal im Vektor vor. Die klonierten cDNAs haben alle dieselbe Orientierung im Vektor. Die Erkennungssequenz E2 liegt am 5'-Ende der cDNA, die Erkennungssequenz E1 am 3'-Ende der cDNA.
2. Unmittelbar neben der Klonierungsstelle des 3'-Endes der cDNA (E1) befindet sich eine Erkennungsstelle für ein selten schneidendes Restriktionsenzym (E3). Alternativ kann die 3 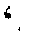 -Klonierungsstelle E1 selber von einem solchen Enzym erkannt werden. Die Erkennungsstelle E3 kommt im allgemeinen nur einmal im Vektor vor. Sie dient dazu, den Vektor definiert aufzuschneiden und markieren zu können, ohne die cDNA zu zerschneiden.
3. Innerhalb einer kurzen Distanz (kleiner als die Distanz von der Schnittstelle E3 zum ersten nicht A Nukleotids des 3'-Endes der cDNA, vorzugsweise kleiner als 30 Basenpaare) befinden sich mindestens zwei Erkennungsstellen für Restriktionsenzyme, die die cDNA häufig schneiden (Region B, Erkennungsstellen E4, E5 und E6). Diese dienen dazu, die cDNA definiert zerschneiden zu können, ohne daß gleichzeitig ein weiteres markiertes Fragment entsteht, das eine vergleichbare Größe hat. Im Beispiel gemäß Abb. 3 sind alle E3-E5-Fragmente, die das 3 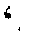 -cDNA-Ende enthalten, größer als das E3-E5-Fragment des Vektors. Das gleiche gilt für die E3-E4- und E3-E6-Fragmente.
4. Unmittelbar im Anschluß an die 5'-Klonierungsstelle befindet sich eine lange, vorzugsweise ein größer als 1000 Nukleotide-langer Bereich (Region A), der keine Erkennungsstellen für die bisher beschriebenen Restriktionsenzyme enthält (Enzyme E1, E2, E3, E4, E5 und E6). Dieser Bereich verleiht markierten Fragmenten aus leeren Vektoren (ohne cDNA-Insert) oder Vektoren mit unvollständigem bzw. kurzem cDNA-Insert (ohne Erkennungsstellen für Restriktionsenzyme E3, E4, E5) eine Mindestgröße. Diese Mindestgröße vermeidet im wesentlichen ihre Detektion im Bereich von vorzugsweise 30 bis 1000 Basenpaaren (siehe Abbildung 4). In diesem Bereich können die markierten Fragmente der meisten Gene detektiert werden.
5. Einen Selektionsmarker und einen Replikationsorigin.

Der erfindungsgemäße Vektor kann gemäß dem Fachmann bekannten Standardklonierungsmethoden hergestellt werden. Eine Möglichkeit der Herstellung ist in Beispiel 1 angegeben.

Daher bezieht sich die vorliegende Erfindung auch auf die Herstellung des erfindungsgemäßen Vektors durch Kombination der einzelnen Komponenten des Vektors, insbesondere durch gentechnische Kombination der einzelnen Komponenten.

Die wesentlichen Vorteile des erfindungsgemäßen Klonierungsvektors sind, daß eine gezielte Markierung des 3 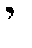 -Endes der cDNA ermöglicht wird und zudem die Eindeutigkeit der Zuordnung zu einem Gen auch dann noch gegeben ist, wenn viele, z. B. bis zu ca. 200 cDNA-Klone simultan analysiert werden. Hierdurch wird eine sehr schnelle Analyse der Genexpression ermöglicht.

Ein anderer Gegenstand der vorliegenden Erfindung ist daher ein Verfahren zur Identifizierung einer Nukleinsäure enthaltend folgende Schritte:
(1) Klonieren einer gegebenenfalls in einer Nukleinsäurepopulation vorhandenen Nukleinsäure in einen erfindungsgemäßen Klonierungsvektor, wobei die Orientierung der Nukleinsäure in dem Klonierungsvektor festgelegt ist,
(2) Hydrolyse mit einer Restriktionsendonuclease, die in Nukleinsäuren selten vorhandene Schnittstellen erkennt,
(3) Aufteilen des gemäß Schritt (2) erhaltenen Reaktionsansatzes in mehrere Portionen,
(4) gegebenenfalls Markieren eines oder beider Enden der gemäß Schritt (3) portionierten Nukleinsäure,
(5) Hydrolyse einer Portion mit einer Restriktionsendonuclease, die in Nukleinsäuren häufig vorhandene Schnittstellen erkennt,
(6) Hydrolyse einer anderen Portion mit einer anderen Restriktionsendonuclease, die in Nukleinsäuren häufig vorhandene Schnittstellen erkennt,
(7) Auftrennen der portionierten Nukleinsäuren, und
(8) Analysieren der aufgetrennten Nukleinsäuren.

Üblicherweise beginnt das Verfahren ausgehend von mRNA, das beispielsweise aus Zellen bzw. Gewebe gewonnen wurde, mit der cDNA-Synthese nach Standardprotokollen. Dabei wird beispielsweise durch die Wahl eines Primergemisches und der Bedingungen für die Erststrangsynthese gewährleistet, daß die Synthese der cDNA an einer festgelegten Position am 3'-Ende der mRNA startet. Danach erfolgt die Insertion der cDNAs in identischer Orientierung in den erfindungsgemäßen Klonierungsvektor (siehe Abb. 2).

Wie oben bereits erwähnt, ermöglicht der Klonierungsvektor in dem erfindungsgemäßen Verfahren die gezielte Markierung des 3'-Endes der cDNA. Ein besonders bevorzugter Vektor trägt zusätzlich an den beiden Seiten der Insertionsstelle definierte Bereiche, die zwei Aufgaben erfüllen:
(i) Durch den kurzen Bereich des Vektors, der beispielsweise am 3'-Ende der cDNA liegt, wird gewährleistet, daß markierte Fragmente des Vektors so klein sind ( z. B. <30 Basenpaare), daß sie die Analyse der Fragmente des 3'-Endes der cDNA, welche größer als beispielsweise 30 Basenpaare sind, nicht stören.
(ii) Durch den langen Bereich des Vektors, der beispielsweise am 5'-Ende der cDNA liegt, wird gewährleistet, daß sehr kurze cDNAs, die keine Restriktionsenzymschnittstellen tragen und damit keine definierten Fragmentlängen des 3'-Bereichs ergeben würden, markierte Fragmente erzeugen, die wiederum zu groß sind ( z. B. >1000 bp), um in dem erfindungsgemäßen Verfahren detektiert zu werden.

Daraus ergibt sich ein sogenanntes 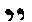 Detektionsfenster" von beispielsweise 30 bis über 1000 Basenpaare, in dem die 3'-Fragmente der meisten cDNAs detektiert werden können.

Nach der Insertion der cDNAs werden die Vektoren nach Transformation in geeigneten Zellen, z. B. prokaryotischen Zellen wie E. coli, vermehrt. Hieraus entstehen sogenannte cDNA-Bibliotheken, die das Expressionsmuster der mRNAs widerspiegeln.

Die Identifizierung der cDNA-Klone erfolgt nach Präparation, Markierung, und Analyse der 3'-Enden durch den Vergleich mit einer Datenbank, die die Fragmentlängen bzw. Fragmentmassen der Restriktionsfragmente des 3'-Bereichs von bekannten cDNAs enthält. Diese Identifizierung ist auch dann noch möglich, wenn Gemische von bis zu ca. 200 cDNA-Klonen gleichzeitig analysiert werden.

Zusätzlich erlaubt der Vergleich mit der Datenbank bekannter Gene, unbekannte Gene in den cDNA-Populationen bzw. -Gemischen zu identifizieren, zu klonieren und wiederum in die Datenbank zu integrieren. Durch dieses Verfahren können somit auch spezifische neue Genbanken aufgebaut werden, die durch das Expressionsmuster der ihr zugrunde gelegten mRNAs charakterisiert sind.

Ein wesentlicher Vorteil des erfindungsgemäßen Verfahrens ist, daß sich sehr viele cDNA-Klone schnell identifizieren lassen, z. B. bis zu 50.000 Klone pro Arbeitskraft pro Woche, wodurch sich die relative Häufigkeit von nahezu allen Genen in der cDNA-Bibliothek und damit ein umfassendes Expressionsmuster der Zellen bzw. des Gewebes, aus dem die mRNAs gewonnen wurden, ermitteln läßt. Durch einen Vergleich von verschiedenen cDNA-Bibliotheken lassen sich so differentiell exprimierte Gene leicht und schnell identifizieren. Das erfindungsgemäße Verfahren ist auch deshalb so vorteilhaft, weil Klone, die keine oder nur unvollständige cDNAs enthalten, von der Analyse ausgeschlossen werden.

In einer bevorzugten Ausführungsform wird der gemäß Schrift (2) erhaltene Reaktionsansatz in mindestens zwei, vorzugsweise drei Portionen aufgeteilt und vorzugsweise werden die einzelnen Portionen von Nukleinsäuren unterschiedlich markiert. In einem weiteren Schritt können die einzelnen Portionen an Nukleinsäuren im Falle einer unterschiedlichen Markierung auch vor dem Auftrennen gemäß Schritt (7) wieder vereinigt werden.

Üblicherweise erfolgt die Analyse der gemäß Schrift (7) aufgetrennten Nukleinsäuren über ihre Größe und/oder Masse, wobei in einem Schritt (8) die Größe und/oder Masse der aufgetrennten Nukleinsäuren mit der Größe und/oder Masse von bekannten Nukleinsäuren verglichen werden kann.

Bei dem erfindungsgemäßen Verfahren ist die kodierende Nukleinsäure im allgemeinen eine sogenannte cDNA, die wie folgt herstellbar ist:
(a) Hybridisieren eines Gemisches aus verschiedenen Primern der Formel (I)
   5' Schnittstelle I-(T)ₙ-V 3' (I),
   wobei Schnittstelle I eine Schnittstelle eines Restriktionsenzyms I, T Thymidin, n eine ganze Zahl von ca. 5-50, vorzugsweise ca. 7-40, insbesondere ca. 7-30, vor allem ca. 10-20 und besonders bevorzugt ca. 15-20, V gleich A (Adenin), G (Guanin) oder C (Cytosin) bedeutet, und das Primer-Gemisch alle Permutationen von V enthält, an eine oder mehrere mRNAs.
(b) Herstellen einer doppelsträngigen cDNA,
(c) gegebenenfalls Anbringen von Linkern, Adaptern, d.h. vorgeschnittenen Linkern, die eine Schnittstelle für ein Restriktionsenzym II enthalten, oder Überhängen, das beispielsweise mittels terminaler Transferase erfolgt, an das 5'- und 3'-Ende der Doppelstrang-cDNA,
(d) Hydrolyse der doppelsträngigen cDNA mit dem Restriktionsenzym I und gegebenenfalls dem Restriktionsenzym II.

Überhänge können beispielsweise poly(A)-, poly(T)-, poly(G)- oder poly(C)-Sequenzen sein.

In einer bevorzugten Ausführungsform enthält das Primer-Gemisch Primer der Formel (II)
5' Schnittstelle I-(T)ₙ-VN 3' (II),
mit N gleich A, G, C oder T, wobei das Primer-Gemisch alle Permutationen von V und N enthält.

Anschließend wird vorzugsweise die gemäß Schrift (d) hydrolysierte doppelsträngige cDNA in einen erfindungsgemäßen Klonierungsvektor mit den Schnittstellen der Restriktionsenzyme I und II kloniert.

Ein weiterer Gegenstand der vorliegenden Erfindung ist daher die Verwendung des erfindungsgemäßen Klonierungsvektors zur Identifizierung von Genen.

Ein anderer Gegenstand der vorliegenden Erfindung ist auch eine Genbank erhältlich durch ein erfindungsgemäßes Verfahren, wobei die erfindungsgemäße Genbank zur Identifizierung von Genen, die anschließend beispielsweise über Sequenzierung charakterisiert werden können, verwendet werden kann.

Das folgende allgemeine Beispiel beschreibt zur Erläuterung der einzelnen Ausführungsformen näher das erfindungsgemäße Verfahren, sowie seine Vorteile und Verwendungsmöglichkeiten:

### Reinigung von mRNA aus Gewebe oder Zellen

RNA wird üblicherweise nach Standardmethoden (siehe z.B. Sambrook et al., 1989, Molecular cloning: A Laboratory Manual, Cold Spring Harbor, Cold Spring Harbor Laboratory Press, New York, Kapitel 7) aus z. B. Gewebe oder Zellen extrahiert und gereinigt. Vorzugsweise wird die RNA in Gegenwart von denaturierenden Agentien wie Guanidiniumchlorid oder Guanidiniumthiocyanat isoliert. Alternativ können auch andere Detergentien und Extraktionsagentien verwendet werden.
Nach der Extraktion der Gesamt-RNA erfolgt im allgemeinen die Isolierung der mRNA. Mit Hilfe von z. B. Oligo-dT-Cellulose oder anderen Chromatographiematerialien, die den polyadenylierten Teil der mRNA binden können, wird die mRNA nach bekannten Methoden gereinigt (Sambrook et al., 1989, supra, Kapitel 7). Alternativ kann die mRNA-Isolierung auch entfallen und das Verfahren mit der Gesamt-RNA durchgeführt werden oder die mRNA kann direkt aus dem Gewebe isoliert werden, ohne zuvor die Gesamt-RNA zu reinigen (z.B. gemäß 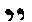 Oligotex direct mRNA Isolation Kit", Qiagen GmbH, Hilden).

### cDNA Synthese mit einem verankertem Primer

Die Erststrang-cDNA-Synthese erfolgt im allgemeinen mit einer Mischung von Primern, die den Poly-A-Schwanz der mRNA und mindestens eine weitere Base der mRNA erkennen (sogenannter verankerter Primer, 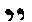 anchored primers" ( siehe z. B. Khan et al., 1991, Nucleic Acids Res. *19*, 1715; Liang et al., 1992, supra; Liang und Pardee, 1992, supra). Hierdurch kann die Synthese der cDNA exakt am Übergang der mRNA-Sequenz zum Poly-A-Schwanz beginnen, wodurch das 3 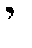 -Ende der cDNA fixiert wird.

Die verankerten Primer bestehen vorzugsweise jeweils aus
(i) einem poly-T Bereich von ca. 5-50, vorzugsweise ca. 7-40, insbesondere ca. 7-30, vor allem ca. 10-20 und ganz besonders bevorzugt ca. 15-20 Thymidin (T)-Resten, der den Poly-A Schwanz der mRNAs erkennt,
(ii) einer Erkennungsstelle eines Restriktionsenzyms 5' zu dem Poly-T Bereich, die der späteren Klonierung dient,
(iii) vorzugsweise einer Verlängerung des 5'-Bereichs mit einer unspezifischen Sequenz, die die Effizienz der Hydrolyse der Erkennungsstelle durch das entsprechende Restriktionsenzym verbessern kann,
(iv) einer der Basen A, G oder C direkt im 3'-Anschluß an den Poly-T Bereich, die die mRNA erkennen und das 3'-Ende der cDNA verankern. Durch die Verwendung einer Mischung aller drei durch die genannten Basen A, G und C gekennzeichneten Primer kannjede beliebige mRNA in der mRNA-Population erkannt werden, und
(v) gegebenenfalls noch ein weiteres Nukleotid einer der Basen A, G, C oder T im 3'-Anschluß der unter (iv) genannten Base, das die mRNA erkennt und eine Verbesserung des spezifischen Synthesestarts der cDNA bewirkt. Durch die Verwendung einer Mischung aller zwölf durch die genannten Basen A, G, C und T gekennzeichneten Primer wird jede beliebige mRNA erkannt, ohne daß eine möglicherweise nicht-gewollte Selektion der mRNAs bewirkt wird.

Der Primer läßt sich beispielsweise durch folgende allgemeine Formel darstellen:
5' Schnittstelle I-(T)ₙ-V 3', (I)
und vorzugsweise durch folgende allgemeine Formel
5' Schnittstelle I-(T)ₙ-VN 3' (II),

wobei Schnittstelle I eine Schnittstelle eines Restriktionsenzyms I bedeutet,
n die oben genannte Bedeutung hat,
V gleich A, G oder C, und gegebenenfalls
N gleich A, G, C oder T, wobei das Primergemisch alle Permutationen von V und N enthält.

Ein typisches Primergemisch mit beispielsweise einer XhoI Schnittstelle bestehend aus 12 verschiedenen Primern hat beispielsweise folgende Formel: 
5‘-GAGAGAGAGA-CTCGAG-TTTTTTTTTTTTTTTTTT-VN-3‘
unspezifisch XhoI Poly-(T) Verankerung

(III),
 mit
V: A, G, oder C, und
N: A, G, C, oder T.

Die optimalen Bedingungen für die Hybridisierung des Primergemisch an die mRNA, die sowohl eine effiziente, als auch an das 3'-Ende fixierte cDNA Synthese ermöglichen, werden vorzugsweise für jedes Primergemisch experimentell ermittelt. Für das Primergemisch gemäß Formel (III) sind diese Bedingungen beispielsweise **5** µg mRNA in 50 µl Hybridisierungspuffer (50 mM Tris-HCl, pH=8,3, 50 mM KCl, 3 mM MgCl₂), der 10 µM Primergemisch enthält, die 5 Minuten lang bei 67° C denaturiert und anschließend 30 Minuten lang bei 38°C hybridisiert werden.

Die Synthese der doppelsträngigen cDNA erfolgt im allgemeinen gemäß Standardmethoden (siehe z.B. Sambrook et al., 1989, supra, Kapitel 8). Vorzugsweise wird die cDNA mit Hilfe einer reversen Transkriptase und einem dNTP Gemisch synthetisiert, wobei eines der Desoxynukleotide methyliert sein kann, um einen späteren Abbau des synthetisierten Strangs zu erschweren bzw. zu verhindern (siehe z.B. Anleitung zum 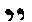 cDNA Synthesis Kit", Stratagene GmbH, Heidelberg, U.S. Patent No. 5, 681, 726).

### Spaltung der cDNA mit Restriktionsenzymen

Die klonierte cDNA wird im allgemeinen mit dem Restriktionsenzym (Restriktionsenzym I) geschnitten, das das 5'-Ende des Primergemischs, das zur cDNA Synthese verwendet wurde, erkennt (z.B. XhoI). Zusätzlich wird im allgemeinen die cDNA mit einem zweiten, andersartigen Enzym (Restriktionsenzym II) gespalten.

Alternativ kann ein vorgeschnittener Adaptor, z.B. ein EcoRI-Adapter, (siehe z. B. Sambrook et al, 1989, supra, Kapitel 8 oder Anleitung zum 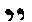 cDNA Synthesis Kit", Stratagene GmbH, Heidelberg) ligiert werden. Desweiteren könnte die cDNA auch mit einem Enzym hydrolysiert werden, das innerhalb der cDNA schneidet. Die Reaktionsbedingungen für die Hydrolyse von DNAs mit Restriktionsendonucleasen sind allgemein bekannt (siehe z.B. Sambrook et al., 1989, supra, Kapitel 5).

### Gerichtete Klonierung der cDNA

Nach der Hydrolyse wird die geschnittene cDNA in einen entsprechend geschnittenen erfindungsgemäßen Klonierungsvektor gemäß Standardmethoden integriert (siehe z.B. Sambrook et al., 1989, supra, Kapitel 1). Üblicherweise erfolgt die Klonierung mit Hilfe der T4 DNA Ligase oder vergleichbaren Enzymen.

### Ausplattieren der cDNA Bibliothek

Die erfindungsgemäßen Klonierungsvektoren, in die die cDNAs integriert worden sind, können beispielsweise verwendet werden, um Zellen zu transformieren und eine cDNA-Bibliothek zu erzeugen. Geeignete Zellen, die die Vektoren mit hoher Effizienz aufnehmen können, und geeignete Transformations- bzw. Transfektionsmethoden sind z.B. in Sambrook et al., 1989, supra, beschrieben. Üblicherweise werden prokaryotische Zellen, vorzugsweise von E. coli, verwendet, z.B. die E. coli Stämme SURE, XL1-Blue MRF 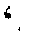 oder XL10-Gold (Stratagene GmbH, Heidelberg).

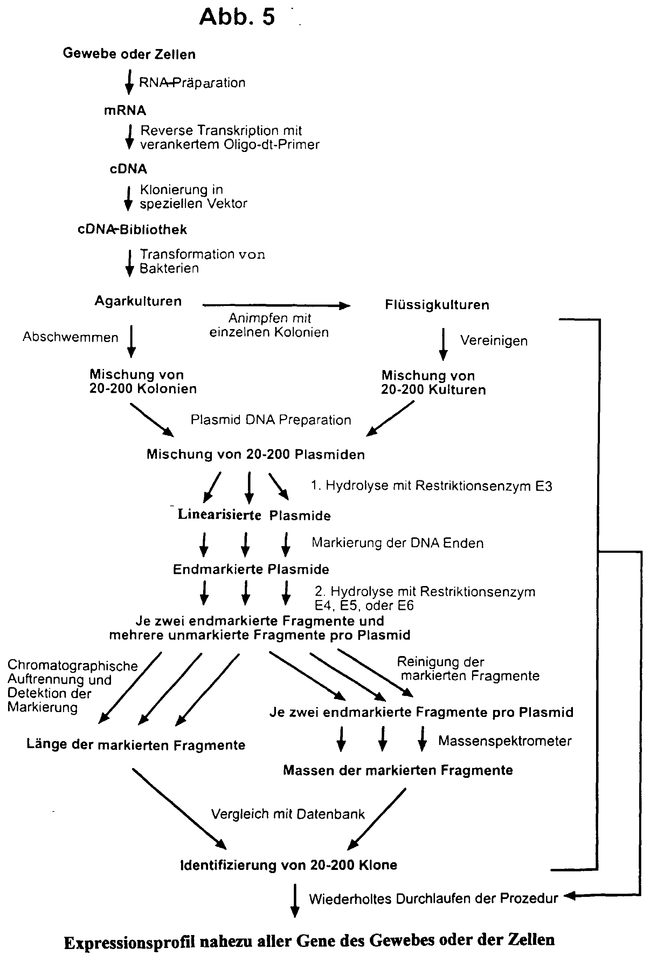

Nach der Transformation wird die Konzentration der resistenten Zellen durch Ausplattieren auf Selektionsmedium und Inkubation unter Wachstumsbedingungen bestimmt (siehe z. B. Sambrook et al., 1989, supra). Danach wird die cDNA-Bank beispielsweise so ausplattiert, daß entweder eine für die Markierung und Detektion geeignete Anzahl von Klonen pro Platte wächst (z.B. 100 Kolonien/Platte, siehe Abbildung 5), oder eine für manuelles oder automatisches Picken von Klonen geeignete Anzahl pro Platte. Im zweiten Fall werden die Kolonien in Flüssigmedium überimpft und unter Wachstumsbedingungen inkubiert.

### Mischen von cDNA Klonen

Eine geeignete Anzahl von Klonen (Kolonien) wird entweder von der Platte geschwemmt und vereinigt oder es werden entsprechend viele Flüssigkulturen vereinigt.

### DNA Plasmidpräperation

Die Plasmid-DNA wird gemäß Standardmethoden (siehe z. B. Sambrook et al., 1989, supra) aus den vereinigten Klonen isoliert und vorzugsweise so gereinigt, daß die nachfolgenden Reaktionen ungestört von Kontaminationen ablaufen können.

### Hydrolyse mit Restriktionsenzym

Die DNA wird beispielsweise aufmindestens zwei, vorzugsweise drei Ansätze verteilt und mit dem Restriktionsenzym geschnitten, das am 3'-Ende der cDNA liegt und cDNAs nur selten schneidet (Enzym E3, siehe Abbildungen 2, 3, 4 und 5).

### Markierung der DNA

Die DNA-Enden, die durch die Hydrolyse mit dem Enzym E3 entstanden sind, werden spezifisch beispielsweise durch ein Isotop (stabil oder radioaktiv), einen Farbstoff oder einen Liganden (z.B. Biotin oder Digoxigenin) markiert. Für diese Markierung bieten sich verschiedene Methoden an:

### A. Enzymatische Markierung (siehe z. B. Sambrook et al., 1989, supra, Kapitel 5)

(i) Ligation eines markierten Oligonukleotids (siehe z. B. Carrano et al., 1989, Genomics *4*, 129-36),
(ii) Kinasereaktion mit einem markiertem Nukleotid-Triphosphat (siehe z. B. Maxam und Gilbert, 1977, Proc. Natl. Acad. Sci. U S A *74*, 560-4),
(iii) DNA-Polymerasereaktion mit einem markiertem Desoxynukleotid-Triphosphat ( 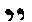 end-labeling", siehe z. B. Sambrook et al., 1989, supra, Kapitel 10), oder
(iv) terminale Transferasereaktion mit einem markiertem Nukleotidtriphosphat oder Desoxynukleotid-Triphosphat (siehe z. B. Cozzarelli et al., 1969, J. Mol. Biol. *45*, 513-31; Roychoudhury et al., 1976, Nucleic Acids Res. *3*, 863-77; Tu und Cohen, 1980, Gene *10*, 177-83).

### B. Chemische Markierung

5'-Markierung über eine Aminohexyl-Phosphorainid-Verbindung (siehe z. B. Chollet und Kawashima, 1985, Nucleic Acids Res. *13*, 1529-41)

### C. Hybridisierung eines markierten Oligonukleotides

(i) Anlagerung eines markierten Oligonukleotides, das eine komplementäre Sequenz zu einem Strang des DNA-Endes hat, wobei die Anlagerung im allgemeinen nach Standardmethoden (siehe z. B. Sambrook et al., 1989, supra) durch einen Denaturierungs-Hybridisierungs -zyklus erfolgt, oder
(ii) Anlagerung eines markierten Oligonukleotides, das in der Lage ist, mit dem DNA-Ende eine Tripelhelix einzugehen (siehe z. B. Francois et al., 1988, Nucleic Acids Res. *16*, 11431-40; Sun et al., 1996, Curr. Opin. Struct. Biol. *6*, 327-33; White et al., 1998, Nature *391*, 468-71).

Mit den nachfolgend beschriebenen Markierungsmethoden, die nur das DNA-Ende markieren, das zur insertierten cDNA hin liegt, kann auf die Region B im erfindungsgemäßen Vektor verzichtet werden. In diesem Fall wird das Vektorfragment nicht markiert. Es kann daher nicht detektiert werden und stört somit die Analyse nicht. Es ist daher auch nicht notwendig, seine Länge mittels Verwendung der Region B auf beispielsweise unter 30 Basenpaare zu begrenzen.

Geeignete Methoden, die spezifisch ein Ende markieren, sind:
(i) Ligation eines markierten Oligonukleotids,
(ii) DNA-Polymerase-Reaktion mit einem markierten Desoxynukleotid-Triphosphat, oder
(iii) Hybridisierung eines markierten Oligonukleotids.

Diese Reaktionen können einseitig erfolgen, wenn die DNA-Enden, die durch die Hydrolyse mit einem Restriktionsenzym entstehen, nicht identisch sind.

Beispiele für die spezifische einseitige Markierung der DNA Enden sind:
(i) Ligation eines markierten Oligonukleotids:
   Durch die Hydrolyse mit Sfil als Enzym E3 (Erkennungssequenz: GGCCNNNNNGGCC) entstehen z.B. folgende DNA Enden: 
5′-GGCCAGGGTGGCC-3′ -> 5′-GGCCAGGG TGGCC-3′
3′-CCGGTCCCACCGG-5′ 3′-CCGGT CCCACCGG-5′.
 Erfolgt nun die Ligation eines markierten, doppelsträngigen Oligonukleotids mit einem 3'-Überhang von drei Cytidinen, so wird nur ein DNA-Ende markiert: 
5'-GGCCAGGG + NNNNNNNNNN
3'-CCGGT CCCNNNNNNNNNNN-Markierung

5'-GGCCAGGGNNNNNNNNNN
3'-CCGGTCCCNNNNNNNNNNN-Markierung
 mit N gleich A, G, C oder T.
   Das andere DNA-Ende wird nicht markiert, da der Überhang nicht kompatibel ist. 
5′-GGCCACCC + NNNNNNNNNNN
3′-CCGGT CCCNNNNNNNNNNNN-Markierung.
(ii) DNA-Polymerase-Reaktion mit einem markierten Desoxynukleotid-Triphosphat: Eine einseitige Markierung durch den Einbau von markierten Desoxynukleotiden erfolgt vorzugsweise nach der Hydrolyse mit Enzymen E3 wie Rsrll (Erkennungsstelle CGGWCCG), die einen 5'-Überhang bildet. 
5′-CGGACCG-3′ -> 5′-CG GACCG-3′
3′-GCCTGGC-5′ 3′-GCCTG GC-5′.
   Die Markierung des einen DNA-Endes erfolgt durch das Auffüllen des Überhangs mit einer DNA-Polymerase und markiertem dATP in Gegenwart von nicht-markierten dCTP, dGTP und dTTP:
   5'-CGG**A**C (unterstrichene Nukleotide: Einbau durch Polymerase, **A**: markiert).
   3'-GCCTG.

   Das andere DNA Ende wird aufgefüllt, aber nicht markiert:
   5'-CGGTC
   3'-GCCAG.
(iii) Hybridisierung eines markierten Oligonukleotids: Durch die Hydrolyse mit Sfil als Enzym E3 (Erkennungssequenz: GGCCNNNNNGGCC) entstehen z.B. folgende DNA-Enden: 
5′-CTCGAGGCCAGGGTGGCCGATCGA-3′ ->
3′-GAGCTCCGGTCCCACCGGCTAGCT-5′

5′-CTCGAGGCCAGGG TGGCCGATCGA-3′
3′-GAGCTCCGGT CCCACCGGCTAGCT-5′.
   Die Markierung erfolgt über die Hybridisierung mit einem markierten Oligonukleotid, das komplementär zu nur einem DNA-Ende ist (Markierung -5'-CCCTGGCCTCGAG):
   5'-CTCGAGGCCAGGG
   3'-GAGCTCCGGTCCC-5'-Markierung.

Für die einseitige Markierung geeignete Restriktionsenzyme sind beispielsweise
(a) Restriktionsendonucleasen mit einer oder mehreren Erkennungssequenzen von 5 Nukleotiden ausgewählt aus AclWI, Alw26I, AlwI, AsuHPI, AvaII, BbvI, BcefI, BinI, Bsbl, BscGI, BselI, BseNI, BsmAI, BsmFI, BspLUllIII, BsrI, BsrSI, Bst7lI, BstF5I, BstNI, CjeI, CjePI, EcoRII, FauI, FinI, FokI, HgaI, HphI, MboII, NciI, PleI, SfaNI, SimI, TauI, TfiI, TseI, Tsp45I, TspRI oder VpallAI,
(b) Restriktionsendonucleasen mit mindestens einer Erkennungssequenz von 6 Nukleotiden ausgewählt aus AccI, AflIII, AvaI, BanI, BanII, BmgI, BsaI, BsiEI, BsiHKAI, BsoBI, Bsp12861, DsaI, EcoO109I, GdiII, Hin4I, MmeI, SfcI, StyI, TatI, TthlllII, BglI, BbsI, BpiI, BpmI, BsaI, BsaMI, BseRI, BsmBI, BsmI, BspMI, Eam1104I, EarI, Eco31I oder Eco57I,
(c) Restriktionsendonucleasen mit Erkennungssequenzen von 6 Nukleotiden, die selten vorkommende Nukleotidkombinationen wie insbesondere CG enthalten, ausgewählt aus BsiI, BsmBI, Bst2BI, Esp3I, oder
(d) Restiktionsendonucleasen mit Erkennungssequenzen von größer als 6 Nukleotiden ausgewählt aus BaeI, PpuMI, RsrII, SanDI, SapI, SexAI, SfiI, I-CeuI, PI-PspI, I-Ppol, PI-TliI oder PI-SceI,

### Hydrolyse mit Restriktionsenzymen

Jeder Ansatz wird im allgemeinen mit mindestens einem häufig schneidenden Enzym geschnitten. Eines dieser Enzyme je Ansatz schneidet den Vektor insbesondere in der Region B (Enzym E4, E5, oder E6). Durch beispielsweise doppelte Hydrolysen mit z. B. E3 und E5 können auch folgende verschiedene Fragmente entstehen (siehe Abbildung 3 und 4):
1. ein markiertes E3-E5 Fragment, das das 3'-Ende der cDNA enthält,
2. ein markiertes, kurzes (<30 bp) E3-E5 Fragment, das Vektorsequenzen enthält, und
3. mehrere unmarkierte E5-E5 Fragmente, die sowohl vom Vektor, als auch von dem cDNA-Insert stammen können.

Sollte der Vektor beispielsweise kein cDNA-Insert enthalten oder ein cDNA Insert enthalten, das keine Erkennungssequenz für das Enzym E5 trägt, so ist Fragment 1 im allgemeinen mindestens 1000 Basenpaare groß, da es die Region A enthält (siehe Abbildung 4).

### Vereinigen der Reaktionsprodukte

Für den Fall, daß die DNA in den verschiedenen Ansätzen unterscheidbar, beispielsweise durch Farbstoffe mit unterschiedlichem Fluoreszensverhalten, markiert worden ist, können die Ansätze im allgemeinen nach Inaktivierung der Restriktionsenzyme wieder vereinigt werden.

### Reinigen der Reaktionsprodukte

Für den Fall einer Markierung der DNA mit radioaktiven Isotopen oder durch Farbstoffe können alle entstehenden DNA-Fragmente z.B. durch Ethanolfällung gereinigt werden. Soll die Analyse im Massenspektrometer erfolgen oder soll nachfolgend eine unspezifische Markierung von DNA, z.B. mit DNA-Farbstoffen wie Ethidiumbromid, erfolgen, so werden im allgemeinen die DNA-Fragmente, die die markierten Enden tragen, aufgereinigt.

### Analyse der Fragmentlängen oder Fragmentmassen

Nach gegebenenfalls erfolgter Reinigung wird üblicherweise die Fragmentlänge und/oder die Fragmentmasse der markierten DNA Fragmente bestimmt. Die Bestimmung der Fragmentlänge kann z.B. nach Markierung mit Fluoreszensfarbstoffen mit einem automatischen DNA-Analyse-System (z.B. ABI Prism™ 377) erfolgen. Die Verfahren hierzu sind in den entsprechenden Anleitungen der Hersteller dieser Systeme eingehend beschrieben. Die Ansätze der verschiedenen Enzyme können auch gleichzeitig analysiert werden, wenn die Markierung mit drei unterschiedlichen Farbstoffen erfolgte. Zusätzlich wird ein mit einem vierten Farbstoff markierter Größenmarker zugegeben, um einen internen Größenstandard zu erhalten. Nach Auftrennen und gleichzeitiger Detektion im DNA-Analyse-System kann mittels einer geeigneten Software (z.B. ABI GeneScan), die die Fluoreszenzsignale der Ansätze mit den Signalen des Markers vergleicht, die Fragmentgröße aller Signale bestimmt und gespeichert werden.

Wurde die DNA radioaktiv markiert oder erfolgte die Markierung mit nur einem Farbstoff wird im allgemeinen jeder Ansatz einzeln analysiert und es wird im allgemeinen auch ein externer Größenmarker verwendet.

Bei der Analyse im Massenspektrometer (MALDI oder ES, siehe z. B. Fu et al., 1998, Nature Biotech., 16, 381-4; U.S. Patent No. 5,627,369, U.S. Patent No. 5,716,825, U.S. Patent No. 5,691,141) werden die Ansätze im allgemeinen ebenfalls einzeln analysiert.

### Auswertung

Die ermittelten Fragmentlängen bzw. -Massen können anschließend mit einer Datenbank verglichen werden. In dieser Datenbank sollten die Längen bzw. Massen der Restriktionsfragmente vom 3'-Ende bekannter cDNAs gespeichert sein. Diese Datenbank kann für bekannte Gene wie folgt angelegt werden:

Ausgehend von der Sequenz wird im allgemeinen die Distanz der Erkennungsstellen der Enzyme E4, E5 und E6 zum 3'-Ende der cDNA (Übergang von der cDNA Sequenz zum Poly-A-Schwanz) ermittelt und die Länge bzw. die Masse dieses DNA-Fragments errechnet. Da die markierten cDNA-Fragmente, die durch das oben beschriebene Verfahren entstehen, noch zusätzlich eine kurzes, definiertes Stück Poly-A-Schwanz und Vektorsequenzen enthalten (Abb. 1), werden die Datenbankeinträge üblicherweise korrigiert. Demzufolge wird zu den Werten in der Datenbank die Länge bzw. die Masse dieser zusätzlichen Sequenz hinzugerechnet.

Jeder Durchlauf des Verfahrens mit einem Pool von cDNA-Klonen ergibt mindestens zwei, üblicherweise drei Listen (je eine für jedes der Enzyme E3, E4 und E5) mit Fragmentlängen bzw. -Massen. Die Einträge in den Listen liegen im allgemeinen im Bereich von ca. 30-1000 Basenpaare. Die Anzahl der Einträge in jeder dieser Listen entspricht der Anzahl der cDNA-Klone, die üblicherweise vereinigt wurden, abzüglich der Klone, die kein oder ein nur kurzes bzw. unvollständiges cDNA-Insert enthalten. Jede Liste wird nun mit der Datenbank verglichen. Im cDNA-Pool können nur die cDNAs solcher Gene enthalten gewesen sein, für die es in jeder Liste einen entsprechenden Eintrag gibt. Taucht auch nur in einer Liste eine in der Datenbank gespeicherte Fragmentlänge nicht auf, so war die cDNA des Gens nicht im Pool enthalten. Gibt es für die Fragmentlängen eines Gens in allen Listen einen entsprechenden Eintrag, so war die cDNA des Gens im Pool enthalten.

Mit diesem Vergleich lassen sich die bekannten Gene bestimmen, deren cDNAs im Pool enthalten waren. Sollte für einen Eintrag in einer oder mehreren Listen kein entsprechendes Gen in der Datenbank gefunden werden, so ist davon auszugehen, daß eine cDNA eines unbekanntes Gens im Pool enthalten war. In einem solchen Fall werden die cDNAs des Pools einzeln sequenziert, um das neue Gen zu identifizieren. Nach der Identifizierung des neuen Gens werden seine Daten in die Datenbank aufgenommen. So kann die Datenbank ständig ergänzt und sehr schnell eine nahezu vollständige Abdeckung aller exprimierten Gene erreicht werden.

Üblicherweise werden in jedem Durchlauf des Verfahrens ca. 20-200 cDNA-Klone identifiziert. Die genaue Anzahl hängt im allgemeinen von verschiedenen Bedingungen ab. Zunächst müssen die Signale jedes Klons eindeutig detektierbar sein, das heißt sich deutlich vom Hintergrund abheben. Wird die Markierung der DNA durch eine Ligation eines fluoreszens-markierten Oligonukleotids durchgeführt und erfolgt die Analyse mit einem automatischen DNA-Analyse-System (ABI Prism™ 377, Applied Biosystems), so können üblicherweise 200 Signale eindeutig detektiert werden (siehe Beispiel).

Zudem sollte gewährleistet sein, daß die Zuordnung zu einem Gen eindeutig ist, das heißt eine zufällige Kombination von Fragmentlängen darf nicht zur falsch positiven Identifizierung einer cDNA im Pool führen. Um eine solche falsch positive Identifikation auszuschließen, sollte die Anzahl der gleichzeitig analysierten cDNAs im Pool begrenzt werden. Die maximale Anzahl von Klonen hängt im allgemeinen von der Anzahl der verwendeten Restriktionsenzyme und damit Listen, der Gesamtzahl der exprimierten Gene und der Reproduzierbarkeit und Genauigkeit der Analyse der Fragmentlängen ab. Werden beispielsweise drei verschiedene Restriktionsenzyme verwendet und Säuger-Zellen bzw. - Gewebe mit ca. 20.000 verschiedenen exprimierten Genen mit Hilfe von Fluoreszensmarkierung und automatischen DNA-Analyse-Systemen analysiert, so ergibt sich im allgemeinen eine maximale Anzahl von 100 Klonen.

Werden alle 64 Spuren eines Gels in einem üblichen DNA-Analyse-System genutzt, so können 64x100= 6.400 Klone pro Gellauf identifiziert werden. Bei 10 Gelläufen pro Woche ergibt sich eine Anzahl von 64.000 identifizierten Klonen. Das Massenspektrometer läßt auf Grund der höheren Genauigkeit der Analyse von Fragmentmassen eine noch höhere Anzahl von identifizierten Klonen zu.

Aus diesen Daten kann die relative Häufigkeit der einzelner cDNAs in der cDNA Bibliothek bestimmt werden. Differentiell exprimierte Gene lassen sich aus dem Vergleich der relativen Häufigkeiten in zwei oder mehreren cDNA-Bibliotheken identifizieren. Eine Anzahl von 64.000 Klonen ermöglicht im allgemeinen für die meisten Gene eine genaue statistische Absicherung der Häufigkeiten (U.S. Patent No. 5, 695, 937; Velculescu et al., 1995, supra; Velculescu et al., 1997, supra; Zhang et al, 1997, Science *276*, 1268-72). Sollen auch sehr schwach exprimierte Gene (1 bis 5 Kopien pro Zelle) verglichen werden, sollten üblicherweise bis zu 300.000 Klone analysiert werden (Zhang et al., 1997, supra).

Die folgenden Abbildungen und Beispiele sollen die Erfindung näher beschreiben, ohne sie zu beschränken:

### Beschreibung der Abbildungen:

- Abb. 1: zeigt schematisch die Identifizierung eines cDNA-Klons aufgrund des charakteristischen Abstands von Restriktionsschnittstellen (E4, E5 und E6) zum 3'-Ende der cDNA.
Die Fragmente der cDNA, die durch die Hydrolyse mit den Restriktionsenzymen E4, E5 oder E6 und der Hydrolyse durch das Enzym E3 entstehen, umfassen Teile des 3'-Endes der cDNA, einen definierten Teil des poly-A-Schwanzes, sowie kurze Vektorsequenzen (siehe Doppelpfeile).
- Abb. 2: zeigt nicht maßstabsgetreu schematisch einen erfindungsgemäßen Klonierungsvektor.
E1 und E2 bedeuten Erkennungssequenzen von Restriktionsenzymen, wobei die durch E1 und E2 definierte Klonierungsstelle der cDNA, nur einmal im Vektor vorkommt.
E3 bedeutet eine Erkennungssequenz für ein selten schneidendes Restriktionsenzym, das nur einmal im Vektor vorkommt.
E4, E5, E6 bedeuten Erkennungssequenzen für häufig schneidende Restriktionsenzyme.
Die Striche unter den Kästchen bedeuten Erkennungssequenzen der Restriktionsenzyme E4, E5, oder E6.
Die Striche über den Kästchen bedeuten Erkennungssequenzen für die Restriktionsenzyme E1, E2 oder E3.
cDNA bedeutet eine klonierte cDNA mit definierter Orientierung, wobei das 5'-Ende der cDNA an die Region A und das 3'-Ende an die Region B anschließt.
Region A bedeutet eine Nukleotidsequenz, die größer als 1000 Basenpaare und frei von Erkennungssequenzen für die Restriktionsenzyme E1-E6 ist.
Region B bedeutet eine Nukleotidsequenz, die kleiner als 30 Basenpaare ist und Erkennungsstellen für die Restriktionsenzyme E4, E5 und E6 enthält.
- Abb. 3: zeigt schematisch die Hydrolyse und Markierung eines erfindungsgemäßen Vektors, der eine vollständige cDNA enthält.
Die Bezeichnung der Vektorelemente erfolgte gemäß Abb. 2.
Die durchgezogenen Linien im Vektor bedeuten Hydrolysestellen und die Sterne Markierungen der Nukleinsäure.
- Abb. 4: zeigt schematisch die Hydrolyse und Markierung eines erfindungsgemäßen Vektors, der eine unvollständige cDNA enthält.
Die Bezeichnung der Vektorelemente erfolgte gemäß Abb. 2 und 3.
- Abb. 5: zeigt eine Skizze vom Ablauf des erfindungsgemäßen Verfahrens.

Hierbei werden die Schritte ab der Mischung der Klone bzw. Kulturen mehrfach wiederholt, bis eine genügende Anzahl von Klonen identifiziert wurde. Nach der Präparation der Plasmid-DNA erfolgt das weitere Verfahren wie beschrieben in drei getrennten Ansätzen, wobei die 2. Hydrolyse mit verschiedenen, häufig schneidenden Restriktionsenzymen erfolgt.

### Beispiele

### 1. Beschreibung des Vektors

Ein erfindungsgemäßer Vektor wurde wie folgt nach Standardklonierungsmethoden (Sambrook et al., 1989, supra) konstruiert:

Der Vektor pUC19 (Yanisch-Perron et al., 1985, Gene *33*, 103-19) wurde mit AatII und HindIII geschnitten und das 2170 bp große Fragment, welches das β-Lactamasegen (Ampicillin-Resistenz) und den ColE1 Replikations-Origin enthält, isoliert.

Zwischen die AatII und HindIII Schittstellen wurde folgendes, doppelsträngige synthetische Oligonukleotid eingefügt:

5′-
AGCTTGGCGCGCCGAATTCTATCTCGAGCGGCCGCAGCTGAGATCGTACCCTA
TAGTGAGTCGTATTACGT-3′
3′-
ACCGCGCGGCTTAAGATAGAGCTCGCCGGCGTCGACTCTAGCATGGGATATCA
CTCAGCATAA-5′

Dadurch ergibt sich folgende Anordnung von Erkennungssequenzen und Elementen:
HindIII-AscI-EcoRI-XhoI-NotI-AluI-DdeI-DpnI-RsaI-T7Promotor

Ausgehend von diesem Konstrukt wurden zwei unterschiedliche Vektoren erzeugt:

### Vektor 1:

Die DNA des Bakteriophagen λ wurde mit DdeI und DpnI geschnitten und das 901 bp große Fragment isoliert. Durch Behandlung mit Klenow-Polymerase wurden die DNA-Enden geglättet. Ein doppelsträngiger, phosphorylierter AscI-Linker (5 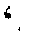 -Pho-AGGCGCGCCT) wurde an die DNA Enden des Fragments ligiert. Es erfolgte eine Hydrolyse mit AseI. Ebenso wurde der Vektor mit dem synthetischen Insert mit AscI hydrolysiert und das DNA-Fragment in den Vektor integriert. Es ergab sich folgende Sequenz an der Integrationsstelle:
GGCGCGCCTTGAGT Insert GGGAAGGCGCGCC,
   wobei der unterstrichene Bereich aus dem 901bp-Fragment stammt.
   Daraus ergeben sich folgende Zuordnungen (vergleiche Abb. 2).
   E1: XhoI; E2: EcoRI; E3: NotI; E4: DdeI; E5: DpnI; E6: RsaI;
   Klonierungsstelle der cDNA: EcoRI-XhoI

Region A: Die Region A geht von der EcoRI Schnittstelle bis zu der ersten DdeI-Schnittstelle, die von dem pUC19 Anteil stammt. Der Bereich ist insgesamt 1558 bp groß und setzt sich aus 634 bp, die aus dem pUC19 Anteil stammen, den 901 bp des DNA-Fragments und 23 bp des synthetischen Inserts zusammen. Die Region trägt keine Erkennungsstellen der Enzyme XhoI, EcoRI, NotI, DdeI, DpnI und RsaI.

Region B: Die Region B geht von der NotI bis zur RsaI Schnittstelle, enthält zusätzlich die Erkennungssequenzen der Enzyme DdeI und DpnI und ist 22 bp lang.

### Vektor 2:

Durch eine Polymerase Ketten Reaktion wurde mit den Primern
5'-CCCCAAGCTTGTGAATATATCGAACAGTCAG-3'und
5'-CCGGCGCGCCTCCCGGTCTTTTCG-3' ein 898 bp DNA Fragment des Bakteriophagen λ amplifiziert und die durch die Primer generierten AscI und HindIII Erkennungssequenzen mit den entsprechenden Enzymen hydrolysiert. Der Vektor mit dem synthetischen Insert wurde ebenfalls mit AscI und HindIII hydrolysiert und das isolierte PCR-Fragment in den Vektor integriert. Es ergab sich folgende Sequenz an der Integrationsstelle:
AAGCTTGTGAA Insert CGGGAGGCGCGCC
   wobei der unterstrichene Bereich aus dem 898 bp-Fragment stammt.
   Daraus ergeben sich folgende Zuordnungen (vergleiche Abb.2)
   E1: XhoI, E2: EcoRI, E3: NotI, E4: DdeI, E5: DpnI, E6: RsaI
   Klonierungsstelle der cDNA: EcoRI-XhoI

Region A: Die Region A geht von der EcoRI Schnittstelle bis zur ersten DdeI-Schnittstelle, die von dem pUC 19 Anteil stammt. Der Bereich ist insgesamt 1546 bp groß und setzt sich aus 634 bp, die aus dem pUC19 Anteil stammen, den 898 bp des PCR-Fragments und 14 bp des synthetischen Inserts zusammen. Die Region trägt keine Erkennungsstellen der Enzyme XhoI, EcoRI, NotI, DdeI, DpnI und RsaI.

Region B: von der NotI bis zur RsaI Schnittstelle, enthält zusätzlich die Erkennungssequenzen der Enzyme DdeI und DpnI und ist 22 bp lang.

### 2. Herstellung einer cDNA Bibliothek

Die cDNA Synthese erfolgte mit dem 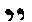 cDNA Synthesis Kit" (U.S. Patent No. 5, 681, 726 Stratagene GmbH, Heidelberg, #200401). Ausgangsmaterial waren 5 µg mRNA. Hierzu wurden 5 µg mRNA in 37,5 µl Wasser 5 Minuten lang bei 67°C denaturiert, auf Eis abgekühlt und mit 5 µl 10 x Erststrangsynthese-Puffer, 3 µl methyliertem Nukleotid Mischung, 1 µl RNase Inhibitor und 3 µg Primergemisches vereinigt. Anstelle des im cDNA Synthesis Kit enthaltenen Primers wurde ein Gemisch von 12 Primern verwendet:
5 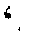 -GAGAGAGAGAGAGAGAGAGAACTAGTCTCGAGTTTTTTTTTTTTTTTTVN-3 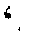

Die Hybridisierung des Primers und die Erststrangsynthese erfolgte nach Zugabe von 1,5 µl MMLV reverser Transcriptase (50 U/µl) für eine Stunde bei 38°C. Danach wurde das Reaktionsgemisch auf Eis abgekühlt und nach Zugabe von 20 µl Zweitstrangsynthese-Puffer, 6 µl Nukleotid-Mischung, 116 µl Wasser, 2 µl RNaseH und 11 µl DNA-Polymerase I erfolgte die Zweitstrangsynthese für 2,5 Stunden bei 16°C. Danach wurden die DNA-Enden der doppelsträngigen cDNA nach Zugabe von 23 µl Nukleotid Mischung und 2 µl Pfu DNA-Polymerase bei 72°C für 30 Minuten geglättet.

Nach Phenol/Chloroform Extraktion und Ethanolfällung erfolgte die Ligation des EcoRI-Adaptors. Dazu wurde die gefällte cDNA in 9 µl der EcoRI-Adaptorlösung aufgelöst und die Ligation erfolgte nach Zugabe von 1 µl des Ligase-Puffers 1 µl 10 mM ATP und 1 µl T4 DNA-Ligase bei 8°C über Nacht. Nach Hitzeinaktivierung der Ligase (30 Minuten bei 70°C) wurden die DNA-Enden des EcoRI-Adapters nach Zugabe von 1 µl Ligase-Puffer, 2 µl 10 µM ATP, 6 µl Wasser und 1 µl T4 Polynukleotid-Kinase für 30 Minuten bei 37°C phosphoryliert. Nach Hitzeinaktierung der Polynukleotid-Kinase (30 Minuten 70°C) wurde die cDNA nach Zugabe von 28 µl XhoI Puffer und 3 µl XhoI für 1,5 Stunden bei 37°C hydrolysiert.

Nach der Hydrolyse mit XhoI erfolgte die Abtrennung überschüssiger Oligonukleotide und sonstiger Verunreinigungen der DNA über Agarosegelelektrophorese in niedrig schmelzender Agarose. Nach der Elektrophorese wurde die cDNA mit Standardmethoden aus der Agarose gereinigt (Sambrook et al., 1989).

Die Integration in den unter Beispiel 1 beschriebenen Vektors erfolgte nach Hydrolyse des Vektors mit XhoI und EcoRI und Reinigung des Vektorfragments. Für die Ligasereaktion mit T4 DNA Ligase (Sambrook et al., 1989) wurde ein Verhältnis von 100 ng cDNA zu 100 ng Vektor in einem Volumen von 5 µl gewählt.

Nach der Ligation wurde die DNA durch Dialyse entsalzt. Die Transformation erfolgte durch Elektroporation in kompetente XL1-Blue MRF 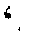 E. coli Zellen (Stratagene GmbH, Heidelberg, #200158) nach Anweisung des Herstellers.

### 3. DNA-Präparation, Hydrolyse, Markierung und Analyse von Klonen

Die cDNA Bibliothek wurde so ausplattiert, daß 55 bis 75 Kolonien pro Selektionsplatte Petrischale mit 10 cm Durchmesser, LB-Agar-Medium mit 100 µl/ml Ampicillin. (Sambrook et al., 1989 Supra) wuchsen. Nach 24 Stunden Inkubation bei 37°C wurden die Kolonien in 1 ml TE-Puffer (10 mM Tris-HCl pH 8,0, 1 mM EDTA, pH 8.0) abgeschwemmt und in einer Mikrozentrifüge pelletiert. Die Bakterien wurden mittels alkalischer Lyse aufgeschlossen, die Plasmid-DNA mit Standardmethoden isoliert (Sainbrook et al., 1989) und in 60 µl TE-Puffer aufgenommen.

Die 1. Hydrolyse mit E3 (NotI) und die Endmarkierung erfolgte in einer gekoppelten Reaktion (Carrano et al., 1989, supra). Die Markierung erfolgte durch die Ligation eines doppelsträngigen, nicht-phosphorylierten Oligonukleotids. Das kürzere Oligonukleotid trägt an seinem 5'-Ende einen Farbstoff (entweder FAM (5-carboxy-fluorescein), TAMRA (N,N,N 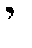 ,N 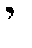 -tetramethyl-6-carboxyrhodamin), oder JOE (2 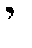 ,7 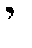 -dimethoxy-4 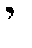 ,5 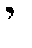 -dichloro-6-carboxy-fluorescein). Das längere Oligonukleotid ist komplementär zu dem kürzeren und hybridisiert so, daß sein 5'-Ende einen Überhang bildet, der komplementär zu dem 5'-Überhang nach Hydrolyse mit dem Restriktionsenzym NotI ist. In der Ligasereaktion wird das 3'-Ende des markierten Oligonukleotids mit dem 5'-Ende der NotI-Schnittstelle kovalent verknüpft. Durch die Wahl der Sequenz des Oligonukleotids wird verhindert, daß nach erfolgter Ligation eine erneute Hydrolyse mit NotI möglich ist, da die NotI Erkennungssequenz nicht regeneriert wird.

### Sequenz der Oligonukleotide:

Markierung -5'-CAGGAGATGCTGTTCGT-3'
3'-TCCTCTACGACAAGCACCGG-5'

Die Plasmid-DNA wurde auf 3 Ansätze mit je 10 µl verteilt. Die Reaktion wurde durch Zugabe von 15 µl Reaktionsmischung gestartet. Die Endkonzentrationen im Reaktionsgemisch waren wie folgt:

20 mM Tris-Acetat, pH 7,9 bei 25°C; 10 mM Magnesium-Acetat; 50 mM Kalium-Acetat; 0,1 µg/µl acetyliertes BSA; 6 mM DTT; 1 mM ATP; 0,16 µM des doppelsträngigen Oligonukleotids; 0,2 Einheiten/µl NotI; 0,04 Einheiten/µl T4 DNA Ligase (Weiss Einheiten).

Die Inkubation erfolgte für 16 Stunden bei 37°C. Nach der Hydrolyse und Markierung erfolgte eine Hitzeinaktivierung der Enzyme bei 65°C für 15 Minuten.

Die 2. Hydrolyse mit den Restriktionsenzymen E4 (DdeI), E5 (DpnI) oder E6 (RsaI) erfolgte durch Zugabe von 20 µl des folgenden Reaktionsgemisches:

20 mM Tris-Acetat, pH=7,9 bei 25°C; 10 mM Magnesium-Acetat; 50 mM Kalium-Acetat; 0,1 µg/µl acetyliertes BSA; 0,1 Einheiten/µl des entsprechenden Enzyms.

Der Ansatz, der mit dem FAM-markiertem Oligonukleotid markiert worden war, wurde mit DdeI hydrolysiert. Im Falle einer Markierung mit TAMRA erfolgte die Hydrolyse mit DpnI und im Falle einer Markierung mit JOE erfolgte die Hydrolyse mit RsaI.

Die Reaktion wurde 3 Stunden bei 37°C inkubiert. Danach wurden die Restriktionsenzyme durch Inkubation bei 95°C für 5 min inaktiviert und die drei Ansätze vereinigt. Die DNA wurde nach Zugabe von 15 µl 3 M Natrium-Acetat und 375 µl Ethanol für 30 Minute bei 20°C präzipitiert (Sambrook et al., 1989). Nach Pelletierung in einer Mikrozentrifuge wurde das Pellet mit 70% Ethanol gewaschen und in 2 µl Auftragspuffer (80% Formamid, 5 mM EDTA, 2 mg/ml Dextran Blau, 10 Vol.-% GeneScan-2500 Rox Größenmarker, Applied Biosystems Produkt Nr. 401100) aufgelöst. Die Ansätze wurden 3 Minuten bei 95°C denaturiert und sofort auf Eis abgekühlt.

Die Auftrennung der Fragmente erfolgte in einem automatischen DNA-Analysesystem (ABI Prism™ 377, Applied Biosystems) auf einem 36 cm langen, 0,2 mm dicken, denaturierenden 4%igen Polyacrylamidgel nach Anleitung des Herstellers (Applied Biosystems).

Die Auswertung der Fragmentlängen erfolgte im Vergleich zu dem Größenmarker (GeneScan-2500 Rox) mit einer entsprechenden Software (GeneScan, Applied Biosystems). Je nach der ursprünglichen Anzahl von Klonen konnten die Größen von bis zu 70 Fragmenten in allen drei Ansätzen ermittelt werden. Die Standardabweichung der Analyse lag bei einer Fragmentlänge bis 500 Basenpaare unter 0,1 Basenpaar, über 500 Basenpaaren bei unter 1,0 Basenpaar.

### 4. Vergleich der Expression des Gens SPR1a in Haut und Leber

Das Gen SPR1a wird bekanntermaßen spezifisch und stark in der Maus-Haut exprimiert (Kartasova et al., 1996, J. Invest. Dermatol. 106, 294-304). Klone dieses Gens treten daher häufig in Haut-cDNA-Bibliotheken auf und nicht bzw. deutlich seltener in cDNA-Bibliotheken anderer Gewebe (z.B. Leber). Daher wurde dieses Gen für die Validierung der Methode ausgewählt.

Aus Haut und Lebergewebe einer Maus wurden mRNAs isoliert und zwei cDNA Pools hergestellt (siehe Beispiel 2). Diese wurden in den unter Beispiel 1 beschriebenen Vektor 2 kloniert und es wurden zwei cDNA Bibliotheken hergestellt. Von diesen wurden wie in Beispiel 3 beschrieben je ca. 5000 Klone analysiert.

Aus der publizierten Sequenz von SPR1a (Kartosova et al., supra) wurden die Längen der 3' cDNA Fragmente, die durch Hydrolyse mit DdeI (77 bp), DpnI (273 bp) und RsaI (703 bp) entstehen, ermittelt. Da die analysierten Fragmente der cDNAs noch zusätzlich definierte Sequenzen des Poly-A Schwanzes, des Vektors und des Markierungs-Oligonukleotids enthalten (zusammen 44 bp), wurden die Fragmentlängen entsprechend korrigiert. Die so errechneten Fragmentlängen für DdeI (121 bp), DpnI (317 bp) und RsaI (747 bp) wurden mit den Daten der Analysen verglichen. Dabei wurde berücksichtigt, daß DNA Fragmente in denaturierenden Polyacrylamidgelen sequenzabhängige und reproduzierbare Abweichung in der Laufgeschwindigkeit zeigen, so daß die im Vergleich zu einem Größenmarker ermittelten Fragmentlängen um ca. 1% von den tatsächlichen Fragmentlängen abweichen können (Frank und Köster, 1979, Nucleic Acids Res. 6, 2069-87). Klone mit zur publizierten Sequenz von SPR1a vergleichbaren Fragmentlängen (DdeI: 120,59±0,04 bp, DpnI: 319,89±0,04 bp, RsaI: 750,80±0,30bp) traten 8mal in der Analyse der Haut-cDNA-Bibliothek auf, jedoch kein mal in der Analyse der Leber-cDNA-Bibliothek. Die Isolierung und Sequenzierung eines dieser Klone bestätigte, daß diese Klone die cDNA von SPR1a enthielten. Aus diesen Daten kann abgeleitet werden, daß ca. 0,16% (8/5000) der mRNAs in der Haut vom SPR1a Gen stammt, während die Häufigkeit in der Leber unter 0,02% liegt, was den oben erwähnten publizierten Daten entspricht.

Vergleichbare Daten konnten für weitere Gene ermittelt werden. So wurde z.B. das Fragmentmuster von Serumalbumin 27mal in der Analyse der Leber-cDNA-Bibliothek ermittelt, jedoch nicht in der Analyse der Haut-cDNA-Bibliothek. Die so ermittelte Häufigkeit (ca. 0,5%) der Serumalbumin mRNA in der Maus-Leber ist konform mit den publizierten Daten zur Expression von Serumalbumin (Sellem et al., 1984, Dev. Biol. 102,51-60).

## Patentansprüche

1. Klonierungsvektor enthaltend
(a) eine Klonierungsstelle, die die Klonierung einer Nukleinsäure in definierter Orientierung erlaubt,
(b) mindestens eine Schnittstelle, die zu der Klonierungsstelle (a) benachbart ist und die in Nukleinsäuren nur selten vorhanden ist,
(c) einen langen Bereich, der an der bezogen auf die Schnittstelle (b) gegenüberliegenden Seite der Klonierungsstelle (a) angeordnet ist, wobei der lange Bereich und der Bereich zwischen der Klonierungsstelle (a) und der Schnittstelle (b) weder die Klonierungsstelle noch mindestens zwei Schnittstellen, die in Nukleinsäuren häufig vorhanden sind, enthält.

2. Klonierungsvektor nach Anspruch 1, dadurch gekennzeichnet, daß an der bezogen auf die Klonierungsstelle (a) gegenüberliegende Seite der Schnittstelle (b) ein kurzer Bereich mit mehreren verschiedenen Schnittstellen, die in Nukleinsäuren häufig und in dem genannten langen Bereich nicht vorhanden sind, angeordnet ist.

3. Klonierungsvektor nach Anspruch 1 oder 2, dadurch gekennzeichnet, daß die Klonierungsstelle zwei verschiedene Schnittstellen enthält.

4. Klonierungsvektor nach einem der Ansprüche 1-3, dadurch gekennzeichnet, daß die Schnittstelle, die in Nukleinsäuren häufig vorhanden ist, unabhängig voneinander eine Stelle ist, die von Restriktionsendonucleasen mit einer Erkennungssequenz von höchstens einschließlich 4 Nukleotiden erkannt werden.

5. Klonierungsvektor nach Anspruch 4, dadurch gekennzeichnet, daß die genannte Schnittstelle ausgewählt ist aus einer Stelle, die von einer der Restriktionsendonucleasen ausgewählt aus AciI, AluI, BfaI, BsaJI, BslI, BscFI, BssKI, BstUI, Cac8I, CfoI, Csp6I, CviJI, DdeI, DpnI, DpnII, FmuI, Fnu4HI, HaeIII, HhaI, HinfI, HinPI, HpaII, MaeII, MaeIII, MboI, MnlI, MseI, MspI, MwoI, NlaIII, NlaIV, RsaI, Sau3AI, Sau96I, ScrFI, TaiI, TaqI, Tsp4CI oder Tsp509I erkannt wird.

6. Klonierungsvektor nach einem der Ansprüche 1-5, dadurch gekennzeichnet, daß die Klonierungs- und die Schnittstelle, die in Nukleinsäuren nur selten vorhanden ist, ausgewählt ist aus einer Schnittstelle, die von Restriktionsendonucleasen mit einer Erkennungssequenz von mindestens einschließlich 5 Nukleotiden, vorzugsweise mindestens einschließlich 6 Nukleotiden, insbesondere mindestens einschließlich 8 Nukleotiden erkannt wird.

7. Klonierungsvektor nach Anspruch 6, dadurch gekennzeichnet, daß die genannte Klonierungsstelle und/oder Schnittstelle ausgewählt ist aus einer Stelle, die von einer der Restriktionsendonucleasen mit einer oder mehreren Erkennungssequenzen von 5 Basen ausgewählt aus AclWI, Alw26I, AlwI, AsuHPI, AvaII, BbvI, BccI, BcefI, BinI, BsbI, BscGI, Bse1I, BseNI, BsmAI, BsmFI, BspLU11III, BsrI, BsrSI, Bst71I, BstF5I, BstNI, CjeI, CjePI, EcoRII, FauI, FinI, FokI, HgaI, HphI, MboII, NciI, PleI, SfaNI, SimI, TauI, TfiI, TseI, Tsp45I, TspRI oder Vpa11AI erkannt wird.

8. Klonierungsvektor nach Anspruch 6, dadurch gekennzeichnet, daß die genannte Klonierungsstelle und /oder Schnittstelle ausgewählt ist aus einer Stelle, die von einer der Restriktionsendonucleasen mit mehreren Erkennungssequenzen von 6 Basen ausgewählt aus AccI, AflIII, ApoI, AvaI, AvaII, BanI, BanII, BmgI, BsaI, BsaHI, BsaWI, BsiEI, BsiHKAI, BsoBI, Bsp1286I, BsrFI, BstYI, DsaI, EaeI, EcoO109I, GdiII, HaeI, HaeII, Hin4I, HincII, MmeI, MslI, MspA1I, NspI, SfcI, StyI, TatI, Tth111II, AatI, Acc113I, Acc65I, AcINI, AfIII, Alw44I, ApaI, ApaLI, AseI, Asp718I, AvrII, BalI, BamHI, BbuI, BbsI, Bc1I, BfrI, BglI, Bg1II, B1nI, BpiI, BpmI, BsaI, BsaMI, BseRI, BsmBI, BsmI, Bsp120I, Bsp1407I, Bsp19I, BspHI, BspLU11I, BspMI, BspTI, BsrGI, Bst1107I, Bst98I, DraI, Eam1104I, EarI, Ecl136II, Eco147I, Eco255I, Eco57I, EcoNI, EcoRI, EcoRV, EcoT22I, HindIII, HpaI, KpnI, MfeI, MscI, NcoI, NdeI, NheI, NsiI, PstI, PvuII, SacI, ScaI, SpeI, SphI, SspI, SstI, StuI oder XbaI erkannt wird.

9. Klonierungsvektor nach Anspruch 6, dadurch gekennzeichnet, daß die genannte Klonierungsstelle und/oder Schnittstelle ausgewählt ist aus einer Stelle, die von einer Restriktionsendonuclease mit Erkennungssequenzen von 6 Nukleotiden, die selten vorkommende Nukleotidkombinationen wie insbesondere CG enthalten, erkannt wird.

10. Klonierungsvektor nach Anspruch 9, dadurch gekennzeichnet, daß die genannte Klonierungsstelle und/oder Schnittstelle ausgewählt ist aus einer Stelle die von einer der Restriktionsendonucleasen AatII, BbeI, BsiI, BsiWI, BsmBI, BspDI, BsrBI, BssHII, Bst2BI, BstBI, ClaI, EagI, EciI, Eco47III, EheI, Esp3I, FspI, KasI, MluI, NarI, NruI, Pfl1108I, PmlI, Psp1406I, PvuI, SacII, SalI, SnaBI oder XhoI erkannt wird.

11. Klonierungsvektor nach Anspruch 6, dadurch gekennzeichnet, daß die genannte Klonierungsstelle und/oder Schnittstelle ausgewählt ist aus einer Stelle, die von einer der Restriktionsendonucleasen mit Erkennungssequenzen von größer als 6 Nukleotiden ausgewählt aus AscI, BaeI, FseI, NotI, PacI, PmeI, PpuMI, RsrII, SanDI. SapI, SexAI, SfiI, SgfI, SgrAI, SrfI, Sse8387I, SwaI, I-CeuI, PI-PspI, I-PpoI, PI-TliI oder PI-SceI, erkannt wird.

12. Klonierungsvektor nach einem der Ansprüche 1-11, dadurch gekennzeichnet, daß der genannte lange Bereich länger ist als die Fragmente, die durch Schneiden mit Restriktionsendonucleasen erhältlich sind, welche häufig vorhandene Schnittstellen erkennen.

13. Klonierungsvektor nach einem der Ansprüche 1-12, dadurch gekennzeichnet, daß der genannte lange Bereich länger als ca. 500 Nukleotide, vorzugsweise länger als ca. 1000 Nukleotide ist.

14. Klonierungsvektor nach einem der Ansprüche 2-13, dadurch gekennzeichnet, daß der genannte kurze Bereich kleiner ist als die Länge der Nukleinsäure, die sich von der Schnittstelle (b) bis zur ersten möglichen Schnittstelle in der einzuklonierenden Nukleinsäure, die in Nukleinsäuren häufig vorhanden ist, vorzugsweise bis zum Anfang des poly(A)-Schwanzes der einzuklonierenden cDNA, erstreckt.

15. Klonierungsvektor nach einem der Ansprüche 2-14, dadurch gekennzeichnet, daß der genannte kurze Bereich kleiner als ca. 100 Nukleotide, vorzugsweise kleiner als ca. 30 Nukleotide ist.

16. Verfahren zur Herstellung eines Klonierungsvektors nach einem der Ansprüche 1-15, dadurch gekennzeichnet, daß die einzelnen Komponenten des Vektors kombiniert werden.

17. Verfahren zur Identifizierung einer Nukleinsäure enthaltend folgende Schritte:
(1) Klonieren einer gegebenenfalls in einer Nukleinsäurepopulation vorhandenen Nukleinsäure in einen Klonierungsvektor gemäß einem der Ansprüche 1-16, wobei die Orientierung der Nukleinsäure in dem Klonierungsvektor festgelegt ist,
(2) Hydrolyse mit einer Restriktionsendonuclease, die in Nukleinsäuren selten vorhandene Schnittstellen erkennt,
(3) Aufteilen des gemäß Schritt (2) erhaltenen Reaktionsansatzes in mehrere Portionen,
(4) gegebenenfalls Markieren eines oder beider Enden der gemäß Schritt (3) portionierten Nukleinsäure,
(5) Hydrolyse einer Portion mit einer Restriktionsendonuclease, die in Nukleinsäure häufig vorhandene Schnittstellen erkennt,
(6) Hydrolyse einer anderen Portion mit einer anderen Restriktionsendonuclease, die in Nukleinsäuren häufig vorhandene Schnittstellen erkennt,
(7) Auftrennen der portionierten Nukleinsäuren, und
(8) Analysieren der aufgetrennten Nukleinsäuren.

18. Verfahren nach Anspruch 17, dadurch gekennzeichnet, daß Schritt (3) vor Schritt (2) durchgeführt wird.

19. Verfahren nach Anspruch 17 oder 18, dadurch gekennzeichnet, daß der gemäß Schritt (2) erhaltene Reaktionsansatz in mindestens zwei, vorzugsweise drei Portionen aufgeteilt wird.

20. Verfahren nach einem der Ansprüche 17-19, dadurch gekennzeichnet, daß die einzelnen Portionen von Nukleinsäuren unterschiedlich markiert werden.

21. Verfahren nach Anspruch 20, dadurch gekennzeichnet, daß in einem weiteren Schritt einzelnen Portionen an Nukleinsäuren vor dem Auftrennen gemäß Schritt (7) vereinigt werden.

22. Verfahren nach einem der Ansprüche 17-21, dadurch gekennzeichnet, daß die Größe und/oder Masse der aufgetrennten Nukleinsäuren gemäß Schritt (7) analysiert wird.

23. Verfahren nach einem der Ansprüche 17-22, dadurch gekennzeichnet, daß in einem weiteren Schritt (8) die Größe und/oder Masse der aufgetrennten Nukleinsäuren mit der Größe und/oder Masse von bekannten Nukleinsäuren verglichen wird.

24. Verfahren nach einem der Ansprüche 17-23, dadurch gekennzeichnet, daß die zu klonierende Nukleinsäure eine cDNA ist.

25. Verfahren nach Anspruch 24, dadurch gekennzeichnet, daß die cDNA durch folgende Schritte charakterisierbar ist:
(a) Hybridisieren eines Gemisches aus verschiedenen Primern der Formel (I)
5' Schnittstelle I-(T)ₙ-V 3' (I),
wobei Schnittstelle I eine Schnittstelle eines Restriktionsenzyms I, n eine ganze Zahl von ca. 5-50, vorzugsweise ca. 7-40, insbesondere ca. 7-30, vor allem ca. 10-20 und besonders bevorzugt ca. 15-20, V gleich A, G oder C, wobei das Primer-Gemisch alle Permutationen von V enthält, an eine oder mehrere mRNAs.
(b) Herstellen einer doppelsträngigen cDNA,
(c) gegebenenfalls Anbringen von Linkem, Adaptern und/oder Überhängen an das 5'- und 3'-Ende der Doppelstrang-cDNA, die eine Schnittstelle für ein Restriktionsenzym II enthalten,
(d) Hydrolyse der doppelsträngigen cDNA mit dem Restriktionsenzym I und gegebenenfalls dem Restriktionsenzym II.

26. Verfahren nach Anspruch 25, dadurch gekennzeichnet, daß das Primer-Gemisch Primer der Formel (II)
5' Schnittstelle I-(T)ₙ-VN 3' (II),
enthält, mit N gleich A, G oder C oder T, wobei das Primer-Gemisch alle Permutationen von V und N enthält.

27. Verfahren nach Anspruch 25 oder 26, dadurch gekennzeichnet, daß bei dem Herstellen der doppeisträngigen cDNA gemäß Schritt (b) ein Primer verwendet wird, der eine Schnittstelle für ein Restriktionsenzym II enthält.

28. Verfahren nach einem der Ansprüche 25-27, dadurch gekennzeichnet, daß die gemäß Schritt (d) hydrolysierte doppeisträngige cDNA in einen Klonierungsvektor mit den Schnittstellen der Restriktionsenzyme I und II kloniert wird.

29. Verwendung des Klonierungsvektors gemäß einem der Ansprüche 1-15 zur Identifizierung von Genen.

30. Genbank erhältlich durch ein Verfahren gemäß einem der Ansprüche 17-28.

31. Verwendung der Genbank gemäß Anspruch 30 zur Identifizierung von Genen.
